Europäisches Patentamt

European Patent Office

Office européen des brevets

(19)

(11) Veröffentlichungsnummer: **0 361 023 B1**

(12) **EUROPÄISCHE PATENTSCHRIFT**

(45) Veröffentlichungstag der Patentschrift: **05.01.94**

(51) Int. Cl.5: **G01F 23/28**, G01F 23/22, A61M 1/00

(21) Anmeldenummer: **89114021.2**

(22) Anmeldetag: **28.07.89**

Die Akte enthält technische Angaben, die nach dem Eingang der Anmeldung eingereicht wurden und die nicht in dieser Patentschrift enthalten sind.

(54) **Füllstandsmessgerät.**

(30) Priorität: **22.08.88 DE 3828441**

(43) Veröffentlichungstag der Anmeldung:
**04.04.90 Patentblatt 90/14**

(45) Bekanntmachung des Hinweises auf die Patenterteilung:
**05.01.94 Patentblatt 94/01**

(84) Benannte Vertragsstaaten:
**DE ES FR GB IT**

(56) Entgegenhaltungen:
DE-A- 3 236 291
US-A- 3 588 859
US-A- 3 939 360
US-A- 4 002 996

(73) Patentinhaber: **Heinze, Werner, Dipl.-Ing.(FH)**
**Kälberweide 1**
**D-86923 Finning(DE)**

(72) Erfinder: **Heinze, Werner, Dipl.-Ing.(FH)**
**Kälberweide 1**
**D-86923 Finning(DE)**

(74) Vertreter: **Herrmann-Trentepohl, Werner,**
**Dipl.-Ing. et al**
**Patentanwälte Herrmann-Trentepohl,**
**Kirschner, Grosse, Bockhorni & Partner**
**Forstenrieder Allee 59**
**D-81476 München (DE)**

EP 0 361 023 B1

**Beschreibung**

Die Erfindung betrifft die Verwendung eines Füllstandmeßgerätes beim Messen des Füllstands von Blut.

Beispielsweise bei herzchirurgischen Operationen muß zwingend ausgeschaltet werden, daß dem Patienten aus dem Blutreservoir Luft anstelle von Blut gepumpt wird, weil bereits geringe Mengen an Luft genügen, um eine fatale Luftembolie herbeizuführen. So kann etwa ein verminderter venöser Fluß aus dem Patienten zu einem raschen Absinken des Niveaus im Blutreservoir eines Oxygenators führen, so daß eine stete Kontrolle des Flüssigkeitsniveaus während der Operation erforderlich ist.

Es ist bereits, ein Unterschreiten eines Mindestfüllstandes über das Gewicht abzulesen, wobei dann jedoch die Waage absolut unempfindlich gegen alle äußeren Einflüsse sein muß, die das Gewicht verändern können, wie beispielsweise Lageänderung der Schläuche, Stöße gegen den Oxygenator und dergleichen mehr. In der Praxis führt ein solches Meßverfahren zu einem außerordentlich aufwendigen mechanischen Gerät, dessen Zuverlässigkeit zu wünschen übrig läßt.

Schließlich sind optische Meßverfahren bekannt, bei denen man den Umstand ausnutzt, daß Blut aufgrund seiner dunklen Farbe Licht absorbiert, so daß sich bei Vorhandensein von Blut die Brechungswinkel an den Grenzflächen ändern. Bei diesen optischen Verfahren ist jedoch nachteilhaft, daß für unterschiedliche Blutreservoirs unterschiedliche mechanische Sensorbefestigungen erforderlich sind. Bei einigen Oxygenatortypen kann das optische Verfahren nur dann angewandt werden, wenn bereits Blut im Oxygenator ist, klare Flüssigkeiten werden hingegen als Luft erkannt.

Jüngere Entwicklungen arbeiten nach dem Ultraschallprinzip. Ein an der Wand des Blutreservoirs angebrachter Ultraschallsensor imitiert nach entsprechender elektrischer Anregung eine Schallwelle durch die - Wandung des Oxygenators in dessen Inneres. Die Frequenz der Ultraschallwelle liegt hierbei im Bereich von ca. 2 MHz. Befindet sich Flüssigkeit im Oxygenator, so kann sich die Welle ausbreite. Trifft sie auf eine Grenzfläche, so wird ein Teil der Welle reflektiert und lauft zurück zum Schallwandler, der mittlerweile als Empfänger geschaltet ist. Da die Amplitude der Schallwelle jedoch auf ihrem Weg durch das Blutreservoir sehr stark abgeschwächt wird, sind sehr empfindliche Verstärker erforderlich, so daß die Ultraschallverfahren enorm störanfällig sind. Ein weiterer Nachteil besteht darin, daß die Ultraschallverfahren nur für bestimmte Kunststoffarten, insbesondere Makrolon anwendbar sind, nicht jedoch für andere Materialien. Ferner können sich Störfaktoren über die Wandstärke ergeben, da bei großen Wandstärken Wandreflexionen auftreten, was zu unsicheren Zuständen führt. Ein weiterer Nachteil ist darin zu sehen, daß zur Ankuppelung des Sensors ein Ultraschallgel zum Zwecke der Energieübertragung auf die Wand des Blutreservoirs verwendet werden muß.

Es sind bereits Verfahren zur Messung von Füllständen im medizinischen Bereich bekannt, die eine Füllstandsänderung durch Änderung des Schwingungsverhaltens lines Oszillators anzeigen, wobei jedoch mehrere Kondensatoren verwendet werden (US-A-4,002,996 und US-A-3,588,859).

Schließlich ist es zur Ermittlung eines Fällzustandes von Flüssigwaschmitteln in Behälten bekannt (DE-A-32 36 291), einen einzelnen Kondensator im Verbindung mit einem Oszillator zu verwerden, wobei dort bei einem Füllzustand oberhalb eines Grenzwertes der Oszillater schwingt und die Oszillatorschwingung abreißt, sobald der Flüssigkeitspegel den Grenzwert unterschreitet.

Aufgabe der Erfindung ist es, ein Füllstandsmeßgerät für Blutreservoire zu verwenden welches weitgehend störunanfällig ist und damit eine sichere Arbeitsweise gewährleistet sowie einfach und kostengünstig herstellbar ist.

Diese Aufgabe wird erfindungsgemäß durch die im Patentanspruch 1 enthaltenen Merkmale gelöst, wobei zweckmäßige Weiterbildungen der Erfindung durch die in den Unteransprüchen enthaltenen Merkmale gekennzeichnet sind.

Die Erfindung macht sich den Umstand zu eigen, daß ein grundsätzlich aus einem Rückkoppler und Verstärker bestehender Oszillator dann schwingfähig ist, wenn zwei Bedingungen erfüllt sind, nämlich

(1)     $kv = 1$

(2)     $\alpha + \beta = n\pi,$

wobei n ganze Zahlen sind.

Hierbei bedeutet v denjenigen Faktor, um den der Verstärker die Eingangsspannung $U_e$ des Oszillators verstärkt. Der Winkel $\alpha$ ist der Wert der Phasenverschiebung der Ausgangsspannung $U_a$, die um den Faktor v größer als die Eingangsspannung $U_e$ ist. Der Rückkoppler des Oszillators erhält als Eingangsspannung die Ausgangsspannung des Verstärkers und schwächt die Eingangsspannung um den Faktor k ab und verschiebt die Phase des Signals um den Winkel $\beta$.

Die Gleichung (1) wird als Amplitudenbedingung bezeichnet. Ein Oszillator schwingt nur dann, wenn der Wert der Amplitudenbedingung größer oder gleich 1 ist. Die Gleichung (2) wird als Phasenbedingung bezeichnet und sie besagt, daß eine Schwingung nur dann zustande kommt, wenn die Ausgangsspannung des Rückkopplers phasengleich mit der Eingangsspannung des Verstärkers ist. Erfindungsgemäß wird an einer beliebigen Stelle der Oszillatorschaltung ein Bauteil eingefügt, das seinen Wert füllstandsabhängig so ändert, daß entweder die Phasen- oder die Amplitudenbedingung nicht mehr gegeben ist, wodurch man ein Gebilde erhält, das je nach Füllstand schwingt oder nicht.

In einem zu Erfindung gehörenden Gerät ist dieses füllstandsabhängig reagierende Bauteil durch einen Dipol gebildet, der vorzugsweise am Ausgang des Rückkopplers angeordnet wird und im übrigen auf die Schwingfrequenz des Oszillators abgeglichen ist. Ist der Füllstand über dem Dipol, so kann dieser Energie in die Flüssigkeit abstrahlen, wodurch sich die Ausgangsspannung des Rückkopplers mindert, so daß die Amplitudenbedingung nicht mehr erfüllt ist, vielmehr das Produkt kv kleiner 1 ist, mithin der Oszillator nicht schwingt, wenn der Füllstand über dem durch den Dipol kontrollierten Sicherheitsstand liegt. Sobald der Flüssigkeitsstand unter das Niveau des Dipols absinkt, ist die Amplitudenbedingung erfüllt und der Oszillator schwingt, was in geeigneter Weise, insbesondere akustisch oder optisch wahrnehmbar gemacht werden kann, so daß einen entsprechende Füllstandsregelung vorgenommen werden kann. Die Erfindung eignet sich insbesondere für Blutreservoire in Behältern aus Kunststoff oder Glas (kein Bleiglas).

Erfindungsgemäß wird kein Dipol, sondern ein Kondensator verwendet, der zweckmäßigerweise in das Rückkoppelnetzwerk der Oszillatorschaltung so eingebaut wird, daß er bestimmend für die Phasenlage des Ausgangssignales ist. Wird er ferner so dimensioniert, daß die Schwingbedingung nur dann nicht erfüllt ist, wenn der Füllstand höher als die durch die Lage des Kondensators bedingte Flüssigkeitshöhe ist, so bewirkt ein Abfallen des Füllstandes unter die durch den Kondensator bedingte Lage des Flüssigkeitsniveaus eine Änderung der Phasenbedingung, so daß die Addition aus den Phasenverschiebungen $\alpha$ und $\beta$ gleich einem ganzzahligen Vielfachen von $\pi$ wird, so daß bei Durchschreiten des durch die Lage des Kondensators bedingten Grenzwert der Oszillator schwingt und mithin eine Füllstandsregulierung vorgenommen werden muß.

Zweckmäßigerweise ist der Kondensator gegen die Außenumgebung isoliert, um eine Beeinflussung infolge von Kondenswasser u. dgl. auszuschließen. Zweckmäßigerweise erfolgt die Abisolierung durch Kunststoff. Als Träger eignet sich jedes Material, welches die Aufnahme des Kondensators sowie dessen Befestigung an der Wand des Blutreservoirs ermöglicht. Der Träger muß hierbei flexibel sein. Es ist zweckmäßig, hierzu ein Material für gedruckte Schaltungen zu verwenden, so daß der Oszillator im Trägermaterial integriert, d.h. insbesondere durch eine gedrückte Schaltung gebildet sein kann. Nach einer Alternative ist der Oszillator abnehmbar ausgebildet und kann auf am Kondensator ausgebildete Steckkontakte aufgesteckt werden.

Zweckmäßigerweise liegt die Betriebsfrequenz im Mikrowellenbereich, was für die Dimensionierung des Kondensators von Vorteil ist.

Insgesamt zeichnet sich das erfindungsgemäße Meßgerät dadurch aus, daß es für alle Kunststoffe verwendbar ist, aber auch für andere Materialien, wie insbesondere Glas und Keramik. Ferner ist das Meßgerät sehr einfach herstellbar, so daß es sich insbesondere als Wegwerfprodukt eignet. Im besonderen Maße zeichnet sich das erfindungsgemäße Meßgerät durch eine Störunempfindlichkeit aus, so daß eine sehr exakte und zuverlässige Füllstandsmessung möglich ist. Durch die Wahl des Betriebsfrequenzbereichs ist eine Beeinflussung infolge von Störfrequenzen ausgeschlossen, die sich aus weiteren im OP-Bereich arbeitenden Geräten ergeben können.

Nachfolgend werden zur Erläuterung Vorrichtungen anhand der Zeichnung beschrieben, die nicht unter die Erfindung fallen:

Fig. 1    eine perspektivische Schemaansicht eines Meßgeräts an einem behälterartigen Blutreservoir,

Fig. 2    eine Ansicht eines Meßgeräts sowie

Fig. 3    eine schematische Ansicht eines weiteren Meßgeräts.

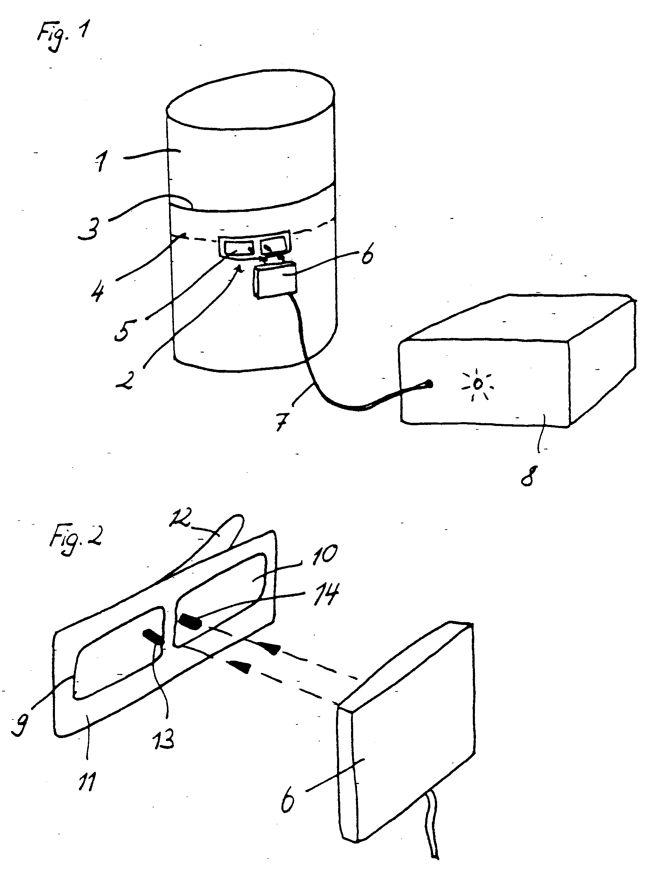

In Fig. 1 ist mit dem Bezugszeichen 1 ein als Blutreservoir dienender Behälter dargestellt, der beispielsweise in Zusammenhang mit einem Oxygenator o. dgl. humanmedizinischem Gerät verwendbar ist. In einem solchen Behälter gilt es eine bestimmte Füllstandshöhe Von Blut aufrechtzuerhalten. Hierzu dient das in Fig. 1 allgemein mit 2 bezeichnete Meßgerät, das in Höhe der zu überwachenden Füllstandshöhe bzw. Flüssigkeitsniveaus angeordnet ist. Das momentan im Behälter vorherrschende Flüssigkeitsniveau ist mit 3 bezeichnet. Die zu überwachende Füllstandshöhe ist schematisch strichliert gekennzeichnet und mit 4 bezeichnet.

Bei dem in Fig. 1 dargestellten Meßgerät 2 ist ein Dipol 5 vorgesehen, an den ein Oszillator 6 anschließbar ist. Der Oszillator 6 ist über eine Leitung 7 mit einem entsprechenden Auswertgerät 8 verbunden.

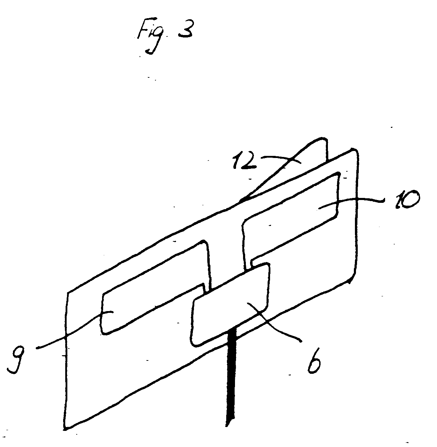

Fig. 2 zeigt das mit einem Dipol ausgerüstete Meßgerät in größerer Deutlichkeit. Danach sind zwei Metallflächen 9 und 10, die den Dipol 5 bilden, nebeneinander auf einem mit 11 bezeichneten Träger angeordnet, mit dem der Dipol an der Wand des Behälters 1 befestigt werden kann. In der dargestellten Vorrichtung ist der Träger an der der Wand des Behälters zugewendeten Seite mit einem Kleber beschichtet, der aus Schutzgründen nach außen hin durch eine abziehbare Folie 12 abgedeckt ist. Auf den den Dipol bildenden Metallflächen 9 und 10 sind Steckkontakte 13 und 14 ausgebildet, auf die der Oszillator 6 aufsteckbar ist.

In der Vorrichtung nach Fig. 3 ist der Oszillator fest, d.h. latent mit den den Dipol bildenden Metallflächen verbunden, insbesondere mit den Metallflächen unter dem Trägermaterial vergossen. Der Träger 11 ist aus einem Material für gedruckte Schaltungen ausgebildet, so daß der Oszillator auf den Träger aufgedruckt sein kann. Der Dipol 5 ist zweckmäßigerweise gegen die Außebumgebung hin abisoliert, wozu der gleichfalls wie der Träger flexibel ausgebildete Dipol mit Kunststoff umgossen ist. Durch diese Maßnahme ist sichergestellt, daß die Funktionsweise nicht durch Kondenswasser u. dgl. gestört wird.

Der Dipol ist auf die Schwingfrequenz des Oszillators abgeglichen. Ist der Füllstand oberhalb der mit 4 bezeichneten Linie, als über dem Dipol, so kann der Dipol Energie in die Flüssigkeit abstrahlen, so daß ein Leistungsverbrauch stattfindet. Dadurch vermindert sich die Ausgangsspannung am Rückkoppler des Oszillalators und die für die Schwingung des Oszillators maßgebliche Amplitudenbedingung von kv = 1 ist nicht erfüllt, so daß der Oszillator nicht schwingt. Mit Durchschreiten des mit 4 bezeichneten Grenzwerts liegt keine Reduktion der Ausgangsspannung des Rückkopplers mehr vor, weil der Dipol keine Energie in die Flüssigkeit abstrahlen kann, so daß nunmehr die Amplitudenbedingung erfüllt ist und damit der Oszillator schwingt. Dies kann akustisch oder optisch wahrnehmbar gemacht werden, so daß eine entsprechende Regelung der Füllstandshöhe vorgenommen werden kann. Es versteht sich von selbst, daß der Dipol in der Oszillatorschaltung integriert ist, wobei der Dipol zweckmäßigerweise am Ausgang des Rückkopplers des Oszillators angeordnet ist.

Wird alternativ zum Dipol erfindungsgemäß ein Kondensator als füllstandsabhängig reagierendes Bauteil verwendet und so in der Oszillatorschaltung integriert, daß der Kondensator bestimmend für die Phasenlage des Ausgangssignales des Oszillators ist und ferner der Kondensator so dimensioniert, daß die Schwingbedingung nur dann nicht erfüllt ist, wenn der Füllstand im Behälter höher als der mit 4 bezeichnete Grenzwert ist, so bewirkt ein Abfallen des Füllstandes unter den mit 4 bezeichneten Wert eine Änderung des für die Schwingbedingung maßgeblichen Werts, d.h. der Phasenbedingung, so daß der Oszillator zu schwingen beginnt, wenn der Füllstand niedriger als der mit 4 bezeichnete Grenzwert ist. Auch dies kann in geeigneter Weise, insbesondere optisch oder akustisch wahrnehmbar gemacht werden, so daß eine entsprechende Füllstandsregelung in Gang gesetzt wird.

Die Betriebsfrequenz des Oszillators beträgt größer gleich 100 MHz, und liegt vorzugsweise im Mikrowellenbereich. Gerade der Mikrowellenbereich ist deswegen geeignet, weil hierdurch sind das füllstandsabhängig reagierende Bauteil gering dimensionieren läßt, etwa die Größe eines Heftpflasters besitzt und somit sehr gut mittels eines geeigneten Trägers auf einer Behälterwand befestigt werden kann.

**Patentansprüche**

1.  Verwendung eines Füllstandsmeßgeräts beim Messen des Füllslands von Blut, mit einem Oszillator und einem füllstandsabhängigen Bauteil, welches auf einem Träger an der Wandung eines Blutreservoirs angeordnet ist und als Teil der Oszillatorschaltung die für die Schwingung des Oszillators maßgeblichen Bedingungen bei Durchschreiten eines voreingestellten Grenzwerts (4) verändert, wenn der Füllstand unter einen vorgegebenen Grenzwert (4) sinkt, wobei die Betriebsfrequenz des Oszillators (6) 100 MHz oder größer ist und das füllstandsabhängige Bauteil durch einen einzelnen Kondensator gebildet ist, wobei die Oszillatorschaltung insgesamt derart abgeglichen ist,' daß der Ostillator (6) zu schwingen beginnt, sobald der Füllstand unter den vorgegebenen Grenzwert (4) sinkt.

2.  Verwendung nach Anspruch 1, wobei der Kondensator gegen die Außenumgebung abisoliert ist.

3.  Verwendung nach Anspruch 1, wobei der Träger (11) flexibel ausgebildet und an der Wandung des Blutreservoirs nach Abzichen einer Folie durch eine Klebeschicht befestigt wird.

4.  Verwendung nach Anspruch 1, wobei daß der Träger (11) aus einem Material für gedruckte Schaltungen hergestellt und der Oszillator (6) im Trägermaterial integriert ist.

5.  Verwendung nach einem der vorhergehenden Ansprüche, wobei die Betriebsfrequenz des Oszillators (6) im Mikrowellenbereich liegt.

**Claims**

1.  The use of a filling level measuring device for measuring the filling level of blood, together with an oscillator and a component reactive to the filling level, which, mounted on a support, is positioned on the wall of a blood reservoir and, as part of the switch mechanism of the oscillator, by exceeding a pre-set threshold value, alters the determining factors governing the vibrations of the oscillator if the filling level sinks below a predetermined threshold level (4), whereby the operating frequency of the oscillator (6) is 100 MHz or greater and the component reactive to the filling level comprises one single condenser, whereby the whole switch mechanism of the oscillator is equalized in such a way that the oscillator (6) begins to vibrate as soon as the filling level sinks below the pre-determined threshold value (4).

2. The use according to claim 1, whereby the condenser is insulated from the surrounding prevailing conditions.

3. The use according to claim 1, whereby the support (11) is flexible and is attached to the wall of the blood reservoir by a layer of adhesive after removal of a strip of film.

4. The use according to claim 1, whereby the support (11) is made of a material for compression switch-mechanisms and the oscillator (6) is integrated within the material of the support.

5. The use according to one of the preceding claims, whereby the operating frequency of the oscillator (6) lies within micro-wave range.

**Revendications**

1. Utilisation d'un appareil pour mesurer le niveau de remplissage d'un récipient avec du sang qui comprend un oscillateur et un composant dépendant du niveau de remplissage lequel est monté, au moyen d un support, sur la paroi d un réservoir de sang et qui, du fait qu'il fait partie du circuit de l'oscillateur, modifie par passage d'une limite prédéterminée, les paramètres déterminant la fréquence dudit oscillateur (6), quand le niveau de remplissage dudit réservoir descend au-dessous d'une certaine limite (4), la fréquence de travail dudit oscillateur (6) étant égale ou supérieure à 100 MHz et le composant influencé par le degré de remplissage est constitué par un condensateur unique, le circuit oscillant étant accordé de telle façon que l'oscillateur (6) se met à osciller dès que le degré de remplissage dudit récipient descend au-dessous de la valeur limite (4) prédéterminée.

2. Utilisation selon la revendication 1, dans laquelle ledit condensateur est isolé du milieu ambiant.

3. Utilisation selon la revendication 1 selon laquelle le support (11) est de conception flexible et après enlèvement d'une feuille de protection, il est collé à la paroi du réservoir contenant le sang par une couche de matière adhésive.

4. Utilisation d'un appareil de mesure telle que spécifié dans la revendication 1, selon laquelle le support (11) est constitué d'un matériau utilisé pour fabriquer les circuits imprimés et l'oscillateur (6) est intégré dans ce matériau support.

5. Utilisation telle que spécifié dans l une quelconque des revendications précédentes, selon laquelle la fréquence de travail de l'oscillateur (6) se situe dans le domaine des micro-ondes.

Fig. 1

1

3

4

5

2

6

7

8

Fig. 2

12

10

14

9

13

11

6

Fig. 3